# EUROPÄISCHE PATENTSCHRIFT

(11) **EP 1 392 485 B1**
(45) Veröffentlichungstag und Bekanntmachung des Hinweises auf die Patenterteilung: **19.04.2006**
(21) Anmeldenummer: 02742964.6
(22) Anmeldetag: 08.05.2002
(51) Int. Cl.: B29C 45/00, B29C 47/76, B29K 33/00

(54) **VERFAHREN ZUM SPRITZGIESSEN VON FORMKÖRPERN AUS (METH)ACRYLAT-COPOLYMEREN**
METHOD FOR INJECTION MOULDING MOULDED BODIES CONSISTING OF (METH)ACRYLATE COPOLYMERS
PROCEDE POUR LE MOULAGE PAR INJECTION DE CORPS MOULES EN COPOLYMERES DE (METH)ACRYLATE

(30) Priorität: 05.06.2001 DE 10127134
(43) Veröffentlichungstag der Anmeldung: 03.03.2004
(73) Patentinhaber: Röhm GmbH & Co. KG, 64293 Darmstadt (DE)
(72) Erfinder: PETEREIT, Hans-Ulrich, 64291 Darmstadt (DE); BECKERT, Thomas, 88447 Warthausen (DE); ASSMUS, Manfred, 64404 Bickenbach (DE); HÖSS, Werner, 63150 Heusenstamm (DE); FUCHS, Wolfgang, 64665 Alsbach (DE); SCHIKOWSKY, Hartmut, 64283 Darmstadt (DE)
(86) Internationale Anmeldenummer: PCT/EP2002/005041
(87) Internationale Veröffentlichungsnummer: WO 2002/098625

(56) Entgegenhaltungen:
- EP-A- 0 316 760
- EP-A- 0 727 205
- EP-A- 0 848 960
- WO-A-01/39751
- WO-A-01/43943
- DE-A- 19 961 334
- US-A- 4 705 695

## Beschreibung

Die Erfindung betrifft ein Verfahren zur Herstellung von Formkörpern mittels Spritzguß, die Formkörper selbst.

### Stand der Technik

(Meth)acrylat-Copolymere, die Monomere mit quaternäre Ammoniumgruppen, z. B. Trimethylammoniumethlymethacrylat-Chlorid, enthalten und deren Verwendung für retardierende Arzneimittelüberzüge sind seit langem bekannt (z. B. aus EP-A 181 515 oder aus DE-PS 1 617 751). Die Verarbeitung erfolgt in organischer Lösung oder als wäßrige Dispersion z. B. durch Sprühen auf Arzneimittelkerne oder auch ohne Lösungsmittel in Gegenwart von Fließmitteln durch Aufbringen in der Schmelze (s. EP-A 0 727 205).

EP 0 704 207 A2 beschreibt thermoplastische Kunststoffe für darmsaftlösliche Arzneiumhüllungen. Es handelt sich dabei um Mischpolymerisate aus 16 bis 40 Gew.-% Acryl- oder Methacrylsäure, 30 bis 80 Gew.-% Methylacrylat und 0 bis 40 Gew.-% anderen Alkylestern der Acrylsäure und/oder Methacrylsäure.

Im Beispiel werden entsprechende Mischpolymerisate bei 160 °C aufgeschmolzen und nach Zugabe von 6 Gew.-% Glycerinmonostearat gemischt. Die Mischung wird gebrochen und zu einem Pulver vermahlen.
Das Pulver wird in die Vorkammer eines Spritzgußwerkzeugs gefüllt und bei 170 °C unter einem Druck von 150 bar durch eine 0,5 mm weite Öffnung in den Formhohlraum gespritzt. Nach Abkühlung erhält man blasenfreie, leicht opake, dünnwandige Arzneimittelkapseln. Besondere Maßnahmen zur Entfernung niedrig siedender Bestandteile unmittelbar vor der Spritzgußverarbeitung sind nicht offenbart.

EP 0 727 205 A2 betrifft ein thermoplastisches Überzugs- und Bindemittel für Arzneiformen, das z. B. aus verschiedenen (Meth)acrylatcopolymeren, u.a. auch solchen mit quaternären Aminogruppen, bestehen kann, die mit Fließmitteln formuliert werden, die auch Trennmittel oder Weichmacher sein können. Die Verarbeitungsmöglichkeit in Spritzgussverfahren wird in allgemeiner Form erwähnt.

EP 0 848 960 A2 beschreibt Haft- und Bindemittel für dermale oder transdermale Anwendungen. Es handelt sich dabei um Mischungen aus (Meth)acrylatcopolymeren, die hohe Weichmachermengen (25 bis 80 Gew.-%) enthalten müssen. Die Bindemittel können u.a. durch Schmelzverarbeitung hergestellt werden. Eine Spritzgussverarbeitung ist nicht vorgesehen.

EP 0 316 760 A2 beschreibt ein Verfahren und eine Vorrichtung zur Herstellung von thermoplastischen Kunststoffen. Das Verfahren wird vorzugsweise zur Durchführung exothermer Polymerisationsreaktionen im einem Polymerisationsextruder angewendet.

### Aufgabe und Lösung

Es wurde als Aufgabe gesehen, ein Verfahren bereitzustellen, daß es erlaubt, die bekannten (Meth)acrylat-Copolymere, die Monomere mit quaternäre Ammoniumgruppen enthalten im Spritzgußverfahren zu verarbeiten. Auf diese Weise sollen Formkörper erhalten werden, die retardierende Eigenschaften aufweisen und hohen mechanischen Anforderungen genügen und daher z. B. als Kapseln (Steckkapseln), die als Behältnisse für pelletierte pharmazeutische Wirkstoffe dienen, verwendet werden können.

Die Aufgabe wird gelöst durch ein
Verfahren zur Herstellung von Formkörpern mittels Spritzguß gemäß dem Anspruch 1
mit den Verfahrensschritten
a) Herstellen einer mischung, enthaltend zumindest ein (meth)acrylat copolymer, einen weichmacher und ein Trockenstellmittel und/oder ein Treinnmittel durch Aufschmelzen und Mischen eines (Meth)acrylat-Copolymeren, das sich aus 85 bis 98 Gew.-% radikalisch polymerisierten C1- bis C4-Alkylestern der Acryl- oder der Methacrylsäure und 15 bis 2 Gew.-% (Meth)acrylatMonomeren mit einer quaternären Ammoniumgruppe im Alkylrest zusammensetzt, mit 10 bis 25 Gew.-% eines Weichmachers, sowie 10 bis 50 Gew.-% eines Trockenstellmittel und/oder 0,1 bis 3 Gew.-% eines Trennmittels,
b) Entgasen der Mischung bei Temperaturen von mindestens 120 °C, wodurch der Gehalt der niedrigsiedenden Bestandteile mit einem Dampfdruck mindestens 1,9 bar bei 120 °C auf höchstens 0,5 Gew.-% gesenkt wird und
c) Einspritzen der entgasten Mischung bei einer Temperatur von 80 bis 160 °C in die Form einer Spritzgußanlage und Entnahme des erhaltenen Formkörpers aus der Form.

Mittels des erfindungsgemäßen Verfahren sind die spritzgegossenen Formkörper gemäß dem Anspruch 4, die hohen mechanischen Anforderungen genügen, erhältlich.

### Ausführung der Erfindung

Das erfindungsgemäße Verfahren zur Herstellung von Formkörpern mittels Spritzguß gliedert sich in die Verfahrensschritte a), b) und c).

### Verfahrensschritt a)

Aufschmelzen und Mischen eines (Meth)acrylat-Copolymeren, das sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammensetzt, mit 10 bis 25 Gew.-% eines Weichmachers, sowie 10 bis 50 Gew.-% eines Trockenstellmittel und/oder 0,1 bis 3 Gew.-% eines Trennmittels und gegebenenfalls weiteren pharmazeutisch üblichen Additiven oder Hilfsstoffen und/oder einem oder mehreren pharmazeutischen Wirkstoffen,

Die Angaben in Gew.-% beziehen sich dabei jeweils auf das (Meth)acrylatCopolymere. Das Aufschmelzen des (Meth)acrylat-Copolymeren, das in Granulat- oder Pulverform vorliegt, erfolgt bevorzugt in einem Extruder bei einer Temperatur von etwa 70 bis 140 °C. Dabei können Trockenstellmittel und/oder Trennmittel und der Weichmacher gleichzeitig oder nacheinander in beliebiger Reihenfolge eingearbeitet werden. Dies gilt auch für gegebenenfalls enthaltene weiteren pharmazeutisch üblichen Hilfsstoffe oder Additive und den gegebenenfalls enthaltenen pharmazeutischen Wirkstoff.

### Das (Meth)acrylat-Copolymer

Entsprechende (Meth)acrylat-Copolymere sind z. B. aus EP-A 181 515 oder aus DE-PS 1 617 751 bekannt. Es handelt sich um unabhängig vom pH-Wert lösliche oder quellbare Polymerisate, die für Arzneimittelüberzügen geeignet sind. Als mögliches Herstellungverfahren ist die Substanzpolymeriation in Gegenwart eines im Monomerengemisch gelösten radikalbildenden Initiators zu nennen. Ebenso kann das Polymerisat auch mittels Lösungs- oder Fällungspolymerisation hergestellt werden. Das Polymerisat kann auf diese Weise in Form eines feinen Pulvers erhalten werden, was bei der Subtanzpolymerisation durch Mahlen, bei Lösungs- und Fällungspolymerisation z. B. durch Sprühtrocknung erreichbar ist.

Das (Meth)acrylat-Copolymer, setzt sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammen.

Bevorzugte C1- bis C4-Alkylestern der Acryl- oder der Methacrylsäure sind Methylacrylat, Ethylacrylat, Butylacrylat, Butylmethacrylat und Methylmethacrylat.

Als (Meth)acrylat Monomer mit quaternären Ammoniumgruppen wird 2-Trimethylammoniumethylmethacrylat-Chlorid besonders bevorzugt.

Ein entsprechendes Copolymer, kann z. B. aus 50 - 70 Gew.-% Methylmethacrylat, 20 - 40 Gew.-% Ethylacrylat und 7 - 2 Gew.-% 2-Trimethylammoniumethylmethacrylat-Chlorid aufgebaut sein.

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (Typ EUDRAGIT® RS).

Ein weiteres geeignetes (Meth)acrylat-Copolymer kann z. B. aus 85 bis weniger als 93 Gew.-% C1- bis C4-Alkylestern der Acryl- oder der Methacrylsäure und mehr als 7 bis 15 Gew.-% (Meth)acrylat Monomeren mit einer quaternären Ammoniumgruppe im Alkylrest aufgebaut sein. Derartige (Meth)acrylat-Monomere sind handelsüblich und werden seit langem für retardierende Überzüge verwendet.

Ein konkret geeignetes Copolymer enthält z. B. 60 Gew.-% Methylmethacrylat, 30 Gew.-% Ethylacrylat und 10 Gew.-% 2-Trimethylammoniumethlymethacrylat-Chlorid (Typ EUDRAGIT® RL).

### Mischungen

Das Meth)acrylat-Copolymer liegt in Mischung mit einem Weichmacher und entweder einem Trockenstellmittel und/oder einem Trennmittel vor. Es können zusätzlich in an sich bekannter Weise können weitere pharmazeutisch übliche Hilfsstoffe und/oder ein pharmazeutischer Wirkstoff enthalten sein.

Der Zusatz von Weichmacher bewirkt eine geringere Sprödigkeit der Formkörper. Dadurch reduziert sich der Anteil gebrochener Formkörper nach Entformung. Ohne Weichmacher liegt der Anteil einwandfrei entnommener Formkörper bei den meisten Mischungen in der Regel bei in etwa 85 %. Mit Weichmacherzusatz kann der Anteil an Entformungsbruch reduziert werden, so daß die Ausbeuten insgesamt meist auf 95 bis 100 % gesteigert werden können.

Als Weichmacher geeignete Stoffe haben in der Regel ein Molekulargewicht zwischen 100 und 20 000 und enthalten eine oder mehrere hydrophile Gruppen im Molekül, z. B. Hydroxyl- , Ester- oder Aminogruppen. Geeignet sind Citrate, Phthalate, Sebacate, Rizinusöl. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 4000 bis 20.000. Bevorzugte Weichmacher sind Tributylcitrat, Triethylcitrat, Acetyltriethylcitrat, Dibutylsebacat und Diethylsebacat. Die Einsatzmengen liegen zwischen 10 und 25, bevorzugt 12 bis 22, besonders bevorzugt 12 bis 18 Gew.-% .-%, bezogen auf das (Meth)acrylat-Copolymere.

### Trockenstellmittel (Antihaftmittel):

Trockenstellmittel können in der Mischung alleine oder zusammen mit Trennmitteln vorliegen. Trockenstellmittel in der Mischung haben folgende Eigenschaften: Sie verfügen über große spezifische Oberflächen, sind chemisch inert, sind gut rieselfähig und feinteilig. Aufgrund dieser Eigenschaften lassen sie sich vorteilhaft in Schmelzen homogen verteilen und erniedrigen die Klebrigkeit von Polymeren, die als funktionelle Gruppen stark polare Comonomere enthalten. Trockenstellmittel (Antihaftmittel) können in einer Menge können in einer Menge von 1 bis 50, bevorzugt 10 bis 40 Gew.-% bezogen auf das Copolymere hinzugefügt werden.

### Beispiele für Trockenstellmittel sind:

Aluminiumoxid, Magnesiumoxid, Kaolin, Talkum, Kieselsäure (Aerosile), Bariumsulfat, Ruß und Cellulose.

### Trennmittel (Formtrennmittel)

Trennmittel (Formtrennmittel) können in der Mischung alleine oder zusammen mit Trockenstellmitteln vorliegen. Trennmittel (Formtrennmittel) müssen in einer Menge können in einer Menge von 0,1 bis 3, bevorzugt 0,2 bis 2,5 Gew.-% bezogen auf das Copolymere hinzugefügt werden.

Im Gegensatz zu Trockenstellmitteln haben Formtrennmittel die Eigenschaft, die Klebkraft zwischen dem Formteilen und der Werkzeugoberfläche, in dem das Formteil hergestellt wird, zu reduzieren. Dadurch wird es möglich, Formteile herzustellen, die nicht zerbrochen und geometrisch nicht deformiert sind. Formtrennmittel sind meist teilverträglich oder unverträglich mit den Polymeren, in denen sie besonders wirksam sind. Durch die Teil- bzw. Unverträglichkeit tritt beim Einspritzen der Schmelze in den Formhohlraum eine Migration in die Grenzfläche des Überganges zwischen Werkzeugwandung und Formteil auf.
Damit Formtrennmittel besonders vorteilhaft migrieren können, soll der Schmelzpunkt des Formtrennmittels 20°C bis 100°C unterhalb der Verarbeitungstemperatur des Polymeren liegen.

### Beispiele für Trennmittel (Formtrennmittel) sind:

Ester von Fettsäuren oder Fettsäureamide , aliphatische, langkettige Carbonsäuren, Fettalkohole sowie deren Ester, Montan- oder Paraffinwachse und Metallseifen, insbesondere zu nennen sind Glycerolmonostearat, Stearylalkohol, Glycerolbehensäureester, Cetylalkohol, Palmitinsäure, Stearinsäure, Kanaubawachs, Bienenwachs etc..

### Additive oder Hilfsstoffe

Die Mischung kann 0 bis 100 Gew.-% von in der pharmazeutisch üblichen Additiven oder Hilfsstoffe bezogen auf das (Meth)acrylat-Copolymere enthalten.

Hier sind z. B, Stabilisatoren, Farbstoffe, Antioxidantien, Netzmittel, Pigmente, Glanzmittel etc. zu nennen. Sie dienen vor allem als Verarbeitungshilfsmittel und sollen ein sicheres und reproduzierbares Herstellungsverfahren sowie gute Langzeitlagerstabilität gewährleisten werden kann..

Als weitere Hilfsstoffe im Sinne der Erfindung sind z. B. auch Polymere zu vertsehen. Die Mischung kann 0 bis 20 Gew.-% eines weiteren Polymeren oder Copolymeren bezogen auf auf das (Meth)acrylat-Copolymere enthalten.

Zur Steuerung der Wirkstoffabgabe kann es im Einzelfall vorteilhaft sein, weitere Polymere zuzumischen. Der Anteil weiterer Polymere an der Mischung beträgt jedoch nicht mehr als 20 Gew.-%, bevorzugt höchstens 10 Gew.-%, insbesondere 0 - 5 Gew.-% .-%, bezogen auf das (Meth)acrylat-Copolymere, beträgt.

Beispiele für solche weiteren Polymere sind: Polyvinylpyrolidone, Polyvinylalkohole, kationische (Meth)acrylat-Copolymere aus Methylmethacrylat und/oder Ethylacrylat und 2-Dimethylaminoethylmethacrylat (EUDRAGIT® E100), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT® NE 30 D), Copolymere aus Methylmethacrylat und Butylmethacrylat (PLASTOID® B).

Geeignet sind auch anionische (Meth)acrylat-Copolymere, die zu 40 bis 100, bevorzugt zu 45 bis 99, insbesondere zu 85 bis 95 Gew.-% aus radikalisch polymerisierten C₁- bis C₄-Alkylestern der Acryl- oder der Methacrylsäure bestehen und bis 60, bevorzugt 1 bis 55, insbesondere 5 bis 15 Gew.-% (Meth)acrylat-Monomere mit einer anionischen Gruppe im Alkylrest enthalten.

Geeignet sind z. B. neutrale (Meth)acrylat Copolymere aus 20 bis 40 Gew.-% Ethylacrylat und 60 bis 80 Gew.-% Methylmethacrylat (Typ EUDRAGIT® NE).

Weiterhin geeignet sind anionische (Meth)acrylat Copolymere aus 40 bis 60, Gew.-% Methacrylsäure und 60 bis 40 Gew.-% Methylmethacrylat oder 60 bis 40 Gew.-% Ethylacrylat (Typen EUDRAGIT® L oder EUDRAGIT® L100-55).

Ebenso geeignet sind anionische (Meth)acrylat Copolymere aus 20 bis 40 Gew.-% Methacrylsäure und 80 bis 60 Gew.-% Methylmethacrylat (Typ EUDRAGIT® S).

Besonders gut geeignet sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS).

### Pharmazeutischer Wirkstoff

Die Mischung kann 0 bis 200 Gew.-% eines oder mehrerer pharmazeutischer Wirkstoffe bezogen auf das (Meth)acrylat-Copolymere enthalten.
Es sollen dabei solche pharmazeutische Wirkstoffe eingesetzt werden, die sich bei Verarbeitungstemperatur nicht zersetzten.

Die im Sinne der Erfindung eingesetzten Arzneistoffe (pharmazeutischer Wirkstoffe) sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichendeStabilität sowie Penetrationsfähigkeit durch die Haut besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer,
Broncholytika/Antiasthmatika, Cholinergika, Corticoide (interna),
Dermatika, Diuretika, Enzyminhibitoren, Enzympräparate und Transportproteine,
Expectorantien, Geriatrika, Gichtmittel, Grippemittel,
Hormone und deren Hemmstoffe, Hypnotika/Sedativa, Kardiaka, Lipidsenker,
Nebenschilddrüsenhormone/Calciumstoffwechselregulatoren,
Psychopharmaka, Sexualhormone und ihre Hemmstoffe,
Spasmolytika, Sympatholytika, Sympathomimetika, Vitamine,
Wundbehandlungsmittel, Zytostatika.

### Verfahrensschritt b)

Das (Meth)acrylat-Copolymer weist vor der Verarbeitung praktisch immer einen Gehalt an niedrigsiedenden Bestandteilen mit einem Dampfdruck von mindestens 1,9 bar bei 120 °C von über 1 Gew.-%, meist um die 2 Gew.-%, auf. Bei den niedrigsiedenden Bestandteilen handelt es sich in der Hauptsache um Wasser, das aus der Luftfeuchtigkeit angezogen wird.

Der Verfahrensschritt b) betrifft das Entgasen der Mischung aus Verfahrensschritt a) bei Temperaturen von mindestens 120 °C, bevorzugt 125 bis 155 °C, besonders bevorzugt 130 bis 140 °C, wodurch der Gehalt der niedrigsiedenden Bestandteile mit einem Dampfdruck mindestens 1,9 bar bei 120 °C auf höchstens 0,5, bevorzugt höchstens 0,2 Gew.-% , besonders bevorzugt höchstens 0,1 Gew.-% gesenkt wird. Dadurch kann vermieden werden, daß es während des Spritzgußvorganges in Verfahrensschritt c) zu einer unerwünschten plötzlichen Entgasung kommt, die zu Blasenbildung oder einem Aufschäumen innerhalb des entstehenden Formkörpers führen würde, der dann unbrauchbar wäre.

Da das (Meth)acrylat-Copolymeren eine Glastemperatur im Bereich von 50 °C aufweist, können niedrigsiedende Bestandteile nicht durch einfaches Trocken bei erhöhter Temperatur entfernt werden, das das Copolymer dabei in ungewünschter Weise versintern oder verfilmen würde.

Deshalb wird der Entgasungsschritt b) bevorzugt durch Extrusionstrocknung in einem Extruder mit Entgasungszone oder durch Entgasen in einer Spritzgußanlage mit vorgeschalteter Entgasungsöffnung erfolgt.
Bei der durch Extrusionstrocknung in einem Extruder mit Entgasungszone wird das entgaste Extrudat unmittelbar in die Spritzgußmaschine, bzw in die Spritzgußform eingeleitet. Beim Entgasen in einer Spritzgußanlage mit vorgeschalteter Entgasungsöffnung erfolgt die Entgasung in einer Vorkammer vor dem Einspressen der Kunststoffschmelze in die Spritzgußform.

Die Mischung kann entweder unmittelbar in Schmelzeform einer Spritzgußanlage zugeführt werden oder bevorzugt zunächst abgekühlt und granuliert werden. Die Lagerung des Granulats sollte unter Bedingungen erfolgen, die eine erneute Wasseraufnahme gering halten, also z. B. nur kurzzeitig und/oder unter trockenen Lagerbedingungen.

### Verfahrensschritt c)

Einspritzen der entgasten Mischung bei einer Temperatur von 80 bis 160 °C, bevorzugt 90 bis 150 °C, besonders bevorzugt 115 bis 145 °C in die Form einer Spritzgußanlage und Entnahme des erhaltenen Formkörpers aus der Form. Die Temperaturangabe bezeichnet die maximal erreichte Temperatur in der heißesten Zone der verwendeten Spritzgußantage.

Die thermoplastische Verarbeitung erfolgt in an sich bekannter Weise mittels einer Spritzgußmaschine bei Temperaturen im Bereich von 80 bis 160 °C, insbesondere zwischen 100 °C und 150 °C und bei Drücken von 60 bis 400 bar, bevorzugt 80 bar bis 120 bar.

Die Formtemperatur liegt bei Glastemperaturen der eingesetzten (Meth)acrylat-Copolymeren in Bereich von z. B. 40°C bis 60°C entsprechend niedriger z. B. bei höchstens 30 oder höchstens 20 °C, so daß die vorliegende Mischung bereits nach kurzer Zeit nach dem Einspritzvorgang in der Form erstarrt und der fertige Formkörper entnommen bzw. entformt werden kann.

Die Formkörper können aus der Formhöhlung des Spritzgießwerkzeuges ohne Bruch entformt werden und weisen eine gleichmäßige, kompakte einwandfreie Oberfläche auf. Der Formkörper zeichnet sich durch mechanische Belastbarkeit bzw. Elastizität und Bruchfestigkeit aus.

Er weist insbesondere eine Schlagzähigkeit nach ISO 179 gemessen an Probekörpern von mindestens 15 KJ/m², bevorzugt von mindestens 18 KJ/m², besonders bevorzugt von mindestens 20 KJ/m2 auf.

Die Wärmeformbeständigkeit VST (A10), gemessen an Probekörpern nach ISO 306 liegt in etwa zwischen 30°C und 60°C.

Die erfindungsgemäß erhaltenen Formkörper können z. B. die Form einer Kapsel, den Teil einer Kapsel, z. B. einer Kapselhälfte, oder einer Steckkapsel aufweisen, die als Behältnis für einen pharmazeutischen Wirkstoff dient. Es können z. B. in Bindemitteln enthaltene Wirkstoffe in Form von Pellets eingefüllt werden und hiernach werden die beiden Kapselteile durch Kleben, Verschweißen durch Laser, Ultraschall bzw. Mikrowellen oder mittels Schnappverbindung zusammengefügt.

Nach diesem Verfahren können erfindungsgemäß auch Kapseln aus unterschiedlichem Material (z.B. Gelatine, anhydrolysierte Stärke, HPMC oder andere Methacrylate) miteinander kombiniert werden. Der Formkörper kann somit auch Teil einer Dosiereinheit sein.

Auch andere Formen, wie Tabletten- oder Linsengeometrien sind möglich. Hierbei enthält der Compound, der für das Spritzgießen zum Einsatz kommt bereits den pharmazeutischen Wirkstoff. In der endgültigen Form liegt der Wirkstoff möglichst gleichmäßig verteilt in kristalliner (Solid Dispersion) oder gelöst amorpher Form (Solid Solution) vor

### Formkörper

Aufgrund des Verfahrensschrittes b) weisen, die im Verfahrensschritt c) erhaltenen spritzgegossenen Formkörper zumindest unmittelbar nach Herstellung einen sehr niedrigen Wassergehalt auf. Der nach der Methode "Karl Fischer" an Probekörpern meßbare Wassergehalt liegt im Bereich kleiner 0,5 Gew.-%. Spätere Änderungen in Wassergehalt, etwa durch längere Lagerung der Formkörper in feuchter Atmosphäre, sind für die Erfindung nicht mehr von Relevanz, da ein niedriger Gehalt an niedrigsiedenden Bestandteilen mit einem Dampfdruck von mindestens 1,9 bar bei 120 °C, womit in erster Linie Wasser bezeichnet werden soll, hauptsächlich für die störungsfreie Ausführung des Verfahrensschritts c) erforderlich ist.

Ein Maß für die Qualität des erhaltenen Formkörpers ist die sogenannte Alkalizahl. Die Alkalizahl ist ähnlich wie die Säurezahl definiert. Sie gibt an, wieviel mg Kaliumhydroxid (KOH) den basischen Gruppen in 1 g Polymer äquivalent sind. Sie wird bestimmt durch potentiometrische Titration entsprechend Ph.Eur.2.2.20 "Potentiometric Titration" oder USP<541>. Als Einwaage wird eine Menge genommen, die 1 g eines Copolymeren mit 10 Gew.-% Trimethylammoniumethlymethacrylat-Chlorid entspricht, in einer Mischung aus 96 ml Eisessig und 4 ml gereinigtem Wasser gelöst und mit 0,1 N Perchlorsäure gegen Quecksilber(II)acetat (Zusatz von 5 ml einer 5%igen Lösung in Eisessig) titriert. Bei thermischer Schädigung des Polymeren in der Mischung sinkt die Alkalizahl im Vergleich zum einer nicht thermisch belasteten Mischung.

Unterschiede in der Alkalizahl von mehr als 0,5 können bereits thermische Schädigungen andeuten. Bei solch einer Schädigung ist zu befürchten, daß die retardierenden Eigenschaften in unakzeptabler Weise verändert sind.

Nach dem erfindungsgemäßen Verfahren sind spritzgegossene Formkörper erhältlich, die einen pharmazeutischen Wirkstoff unmittelbar enthalten können oder die z. B. in Form einer Kapsel einen später eingefüllten pharmazeutischen Wirkstoff umschließen können.

Beispiele für zum Einfüllen in die Formkörper (Kapseln) oder auch für die Einarbeitung in die Formkörper geeignete Wirkstoffe sind: Acetylsalicylsäure Ranitidin, Simvastatin, Enalapril, Fluoxetin, Amlodipin, Amoxicillin, Sertalin, Nifidipin, Ciprofloxacin, Acycolvir, Lovastatin,Epoetin, Paroxetin, Captopril, Nabumeton, Granisetron, Cimetidin, Ticarcillin, Triamteren, Hydrochlorothiazid, Verapamil, Paracetamol, Morphinderivate, Topotecan oder der pharmazeutisch verwendeten Salze.

Die erfindungsgemäße Formulierung eignet sich zur Verabreichung grundsätzlich beliebiger pharmazeutischer Wirkstoffe, die vorzugsweise im Darm und/oder Colon freigesetzt werden sollen, und insbesondere solcher, die mit Vorteil in retardierter Form verabreicht werden können, wie Antidiabetika, Analgetika, Antiphlogistika, Antirheumatika, Antihypotonika, Antihypertonika, Psychopharmaka, Tranquilizer, Antiemetika, Muskelrelaxantien, Glucocorticoide, Mittel zur Behandlung von Colitis ulcerosa oder Morbus Crohn, Antiallergika, Antibiotika, Antiepileptika, Antikoagulantia, Antimykotika, Antitussiva, Arteriosklerosemittel, Diuretika, Enzyme, Enzyminhibitoren, Gichtmittel, Hormone und deren Hemmstoffe, Herzglykoside, Immuntherapeutika und Zytokine, Laxantien, Lipidsenker, Migränemittel, Mineralstoffpraparate, Otologika, Parkinsonmittel, Schilddrüsentherapeutika, Spasmolytika, Thrombozytenaggregationshemmer, Vitamine, Zytostatika und Metastasenhemmer, Phytopharmaka, Chemotherapeutika und Aminosäuren.

Beispiele geeigneter Wirkstoffe sind Acarbose, Betarezeptorenblocker, Nichtsteroidale Antirheumatia, Herzglykoside, Acetylsalicylsäure, Virustatika, Aclarubicin, Acyclovir, Cisplatin, Actinomycin, alpha- und beta-Sympatomimetika, (Dmeprazol, Allopurinol, Alprostadil, Prostaglandine, Amantadin, Ambroxol, Amlodipin, Methotrexat, S-Aminosalicylsäure, Amitriptylin, Amoxicillin, Anastrozol, Atenolol, Azathioprin, Balsalazid, Beclomethason, Betahistin, Bezafibrat, Bicalutamid, Diazepam und Diazepamderivate, Budesonid, Bufexamac, Buprenorphin, Methadon, Calciumsalze, Kaliumsalze, Magnesiumsalze, Candesartan, Carbamazepin, Captopril, Cefalosporine, Cetirizin, Chenodeoxycholsäure, Ursodeoxycholsäure, Theophyllin und Theophyllinderivate, Trypsine, Cimetidin, Clarithromycin, Clavulansäure, Clindamycin, Clobutinol, Clonidin, Cotrimoxazol, Codein, Coffein, Vitamin D und Derivate von Vitamin D, Colestyramin, Cromoglicinsäure, Cumarin und Cumarinderivate, Cystein, Cytarabin, Cyclophosphamid, Ciclosporin, Cyproteron, Cytarabin, Dapiprazol, Desogestrel, Desonid, Dihydralazin, Diltiazem, Mutterkornalkaloide, Dimenhydrinat, Dimethylsulfoxid, Dimeticon, Dipyridarnoi, Domperidon und Domperidanderivate, Dopamin, Doxazosin, Doxorubizin, Doxylamin, Dapiprazol, Benzodiazepine, Diclofenac, Glykosidantibiotika, Desipramin, Econazol, ACE-Hemmer, Enalapril, Ephedrin, Epinephrin, Epoetin und Epoetinderivate, Morphinane, Calciumantagonisten, Irinotecan, Modafinil, Orlistat, Peptidantibiotika, Phenytoin, Riluzole, Risedronat, Sildenafil, Topiramat, Makrolidantibiotika, Estrogen und Estrogenderivate, Gestagen und Gestagenderivate, Testosteron und Testosteronderivate, Androgen und Androgenderivate, Ethenzamid, Etofenamat, Etofibrat, Fenofibrat, Etofyllin, Etoposid, Famciclovir, Famotidin, Felodipin, Fenofibrat, Fentanyl, Fenticonazol, Gyrasehemmer, Fluconazol, Fludarabin, Flunarizin, Fluorouracil, Fluoxetin, Flurbiprofen, Ibuprofen, Flutamid, Fluvastatin, Follitropin, Formoterol, Fosfomicin, Furosemid, Fusidinsäure, Gallopamil, Ganciclovir, Gemfibrozil, Gentamicin, Ginkgo, Johanniskraut, Glibenclamid, Harnstoffderivate als orale Antidiabetika, Glucagon, Glucosamin und Glucosaminderivate, Glutathion, Glycerol und Glycerolderivate, Hypothalamushormone, Goserelin, Gyrasehemmer, Guanethidin, Halofantrin, Haloperidol, Heparin und Heparinderivate, Hyaluronsäure, Hydralazin, Hydrochlorothiazid und Hydrochlorothiazidderivate, Salicylate, Hydroxyzin, Idarubicin, Ifosfamid, Imipramin, Indometacin, Indoramin, Insulin, Interferone, Jod und Jodderivate, Isoconazol, Isoprenalin, Glucitol und Glucitolclerivate, ltraconazol, Ketoconazol, Ketoprofen, Ketotifen, Lacidipin, Lansoprazol, Levodopa, Levomethadon, Schilddrüsenhormone, Liponsäure und Liponsäurederivate, Lisinopril, Lisurid, Lofepramin, Lomustin, Loperamid, Loratadin, Maprotilin, Mebendazol, Mebeverin, Meclozin, Mefenaminsäure, Mefloquin, Meloxicam, Mepindolol, Meprobamat,Meropenem, Mesalazin, Mesuximid, Metamizol, Metformin, Methotrexat, Methylphenidat, Methylprednisolon, Metixen, Metoclopramid, Metoprolol, Metronidazol, Mianserin, Miconazol, Minocyclin, Minoxidil, Misoprostol, Mitomycin, Mizolastin, Moexipril, Morphin und Morphinderivate, Nachtkerze, Nalbuphin, Naloxon, Tilidin, Naproxen, Narcotin, Natamycin, Neostigmin, Nicergolin, Nicethamid, Nifedipin, Nifluminsäure, Nimodipin, Nimorazol, Nimustin, Nisoldipin, Adrenalin und Adrenalinderivate, Norfloxacin, Novaminsulfon, Noscapin, Nystatin, Ofloxacin, Olanzapin, Olsalazin, Omeprazol, Omoconazol, Ondansetron, Oxaceprol, Oxacillin, Oxiconazol, Oxymetazolin, Pantoprazol, Paracetamol, Paroxetin, Penciclovir, orale Penicilline, Pentazocin, Pentifyllin, Pentoxifyllin, Perphenazin, Pethidin, Pflanzenextrakte, Phenazon, Pheniramin, Barbitursäurederivate, Phenylbutazon, Phenytoin, Pimozid, Pindolol, Piperazin, Piracetam, Pirenzepin, Piribedil, Piroxicam, Pramipexol, Pravastatin, Prazosin, Procain, Promazin, Propiverin, Propranolol, Propyphenazon, Prostaglandine, Protionamid, Proxyphyllin, Quetiapin, Quinapril, Quinaprilat, Ramipril, Ranitidin, Reproterol, Reserpin, Ribavirin, Rifampicin, Risperidon, Ritonavir, Ropinirol, Roxatidin, Roxithromycin, Ruscogenin, Rutosid und Rutosidderivate, Sabadilla, Salbutamol, Salmeterol, Scopolamin, Selegilin, Sertaconazol, Sertindol, Sertralion, Silikate, Simvastatin, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Taliolol, Tamoxifen, Taurolidin, Tazaroten, Temazepam, Teniposid, Tenoxicam, Terazosin, Terbinafin, Terbutalin, Terfenadin, Terlipressin, Tertatolol, Tetracycline, Tetryzolin, Theobromin, Theophyllin, Butizin, Thiamazol, Phenothiazine, Thiotepa, Tiagabin, Tiaprid, Propionsaurederivate, Ticlopidin, Timolol, Tinidazol, Tioconazol, Tioguanin, Tioxolon, Tiropramid, Tizanidin, Tolazolin, Tolbutamid, Tolcapon, Tolnaftat, Tolperison, Topotecan, Torasemid, Anti6strogene, Tramadol, Tramazolin, Trandolapril, Tranylcypromin, Trapidil, Trazodon, Triamcinolon und Triamcinolonderivate, Triamteren, Trifluperidol, Trifluridin, Trimethoprim, Trimipramin, Tripelennamin, Triprolidin, Trifosfamid, Tromantadin, Trometamol, Tropalpin, Troxerutin, Tulobuterol, Tyramin, Tyrothricin, Urapidil, Ursodeoxycholsäure, Chenodeoxycholsäure, Valaciclovir, Valproinsäure, Vancomycin, Vecuroniumchlorid, Viagra, Venlafaxin, Verapamil, Vidarabin, Vigabatrin, Viloxazin, Vinblastin, Vincamin, Vincristin, Vindesin, Vinorelbin, Vinpocetin, Viquidil, Warfarin, Xantinolnicotinat, Xipamid, Zafirlukast, Zalcitabin, Zidovudin, Zolmitriptan, Zolpidem, Zoplicon, Zotepin und dergleichen.

Beispiele besonders bevorzugter Wirkstoffe sind Analgetika, wie Tramadol oder Morphin, Mittel zur Behandlung von Colitis ulcerosa oder Morbus Crohn, wie 5-Aminosalicylsäure, Corticosteroide, wie Budesonid, Protonenpumpen-Inhibitoren, wie Omeprazol, Virusstatika, wie Acyclovir, Lipidsenker, wie Simvastatin oder Pravastatin, H2-Blocker, wie Ranitidin oder Famotidin, Antibiotika, wie Amoxicillin und/oder Clavulansäure, und ACE-Hemmer, wie Enalapril oder Amlodipin.

Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Gewünschtenfalls können die erfindungsgemässen Zusammensetzungen auch zwei oder mehrere pharmazeutische Wirkstoffe enthalten.

### BEISPIELE

### Vergleichsbeispiel 1: (Temperatur zu hoch)

### Entgasung und Herstellung der Mischung (Compound)

In einen 101 Mischbehälter aus Edelstahl werden 3,25 kg Granulat EUDRAGIT® RL 100 und 1,0 kg Talkum eingewogen und anschließend auf einem Taumelmischer 5 min gemischt.

Die hergestellte Mischung wurde auf einen Doppelschneckenextruder Typ 30.34 (Fa. Leistritz) gegeben, um einen erfindungsgemäßen Compound herzustellen. Die gemessene Schmelzetemperatur betrug 140°C, die Schneckendrehzahl 120 1/min. Nach einer Länge von 50 % der Gesamtlänge der Extruderschnecke wurde durch eine Öffnung in der Zylinderwand Triethylcitrat als Weichmacher über eine Membranpumpe in einer Menge zugegeben, die 15 % bezogen auf das Copolymer entspricht. Nach einer Mischzone für die Homogenisierung der Mischung erfolgte die Entgasung über eine Entgasungs-Öffnung im Extruderzylinder. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt von 0,09 Gew.-% bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden folgende Temperaturen eingestellt: Zone 1 (Einzugszone): 70°C. Zone 2: 120°C, Zone 3: 160°C, Zone 4: 160°C, Zone 5 (Düse): 130°C. Einspritzdruck: 60 bar, Nachdruck: 50 bar, Staudruck: 5 bar. Werkzeugtemperatur: 17°C (gekühlt)

Als Formkörper wurde ein Plättchen 65 x 40 x 1mm spritzgegossen.
Es konnten Plättchen frei von Schlieren mit einwandfreier glatter Oberfläche hergestellt werden. Die Plättchen ließen sich problemlos entformen und sind geometriestabil. Wegen der hohen Temperatur ist jedoch mit Schädigung des Polymeren zu rechnen.

### Vergleichsbeispiel 2: (Kein Weichmacher)

### Entgasung

Die Herstellung erfolgt wie in Beispiel 1, jedoch ohne Zugabe von Triethylcitrat als Weichmacher.

### Spritzguss

Erfolgte wie in Beispiel 1 beschrieben. In Zone 3 und Zone 4 wurden Temperaturen von 120°C eingestellt.
Ergebnis: Es konnten keine geometrisch ausgeformten und gleichmäßigen Formkörper hergestellt werden. Die Ursache ist in der zu geringeren Fließfähigkeit des Polymer EUDRAGIT® RL 100.

### Beispiel 3 (erfindungsgemäß)

### Entgasung und Herstellung des Compounds

Die Herstellung erfolgt wie in Beispiel 1.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 120°C eingestellt. Als Formkörper wurde ein Plättchen 65 x 40 x 1mm spritzgegossen.

Es konnten Plättchen frei von Schlieren mit einwandfreier glatter Oberfläche hergestellt werden. Die Plättchen ließen sich problemlos entformen und sind geometriestabil.

An den erhaltenen Formkörpern wurde die Alkalizahl bestimmt. Die Alkalizahl ist ähnlich wie die Säurezahl definiert. Sie gibt an, wieviel mg Kaliumhydroxid (KOH) den basischen Gruppen in 1 g Polymer äquivalent sind. Sie wird bestimmt durch potentiometrische Titration entsprechend Ph.Eur.2.2.20 "Potentiometric Titration" oder USP<541 >. Als Einwaage wird eine Menge genommen, die 1 g EUDRAGIT® RL 100 entspricht, in einer Mischung aus 96 ml Eisessig und 4 ml gereinigtem Wasser gelöst und mit 0,1 N Perchlorsäure gegen Quecksilber(II)acetat (Zusatz von 5 ml einer 5%igen Lösung in Eisessig) titriert.
Als Ergebnis wurde eine Alkalizahl (mg KOH/g Polymer) von 23.1 erhalten. Im Vergleich einem nicht durch Spritzgießverarbeitung thermisch belasteten Polymer EUDRAGIT® RL 100 zeigt sich ein vergleichbar gutes Ergebnis mit einer Alkalizahl von 22.9.

### Vergleichsbeispiel 4: (kein Trockenstellmittel oder Formtrennmittel)

### Entgasung und Herstellung des Compounds

Über eine gravimetrische Dosiereinrichtung wurde in die Einzugszone des Doppelschneckenextruders 10 kg EUDRAGIT® RL 100 pro h zudosiert. Mit einer Schneckendrehzahl von 120 U/min wurde das Granulat in den Extruder eingezogen und plastifiziert. Die eingestellte Schmelzetemperatur betrug 140°C.

Nach einer Länge von 50 % der Gesamtlänge des Doppelschneckenextruders ist in der Zylinderwandung eine Öffnung eingebracht, über die mittels einer Membranpumpe Triethylcitrat in einer Menge von 20% bezogen auf die Polymermenge zugepumpt wird.

Nach einer Mischzone für die Homogenisierung der Mischung erfolgte die Entgasung über eine weitere Öffnung in der Zylinderwand. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt von 0,1 % bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 140°C eingestellt. Als Formkörper wurde ein Plättchen 65 x 40 x 1 mm spritzgegossen.

Als Formkörper wurde ein Plättchen 65 x 40 x 1 mm spritzgegossen.
Schon nach zwei Schüssen stellte man erhöhte Klebrigkeit der Formlinge fest mit erschwerter Trennung aus der Form fest, so dass der Versuch abgebrochen werden musste.

### Vergleichsbeispiel 5 (Temperatur zu hoch)

### Entgasung und Herstellung des Compounds

Aus EUDRAGIT® RL 100-haltigen, entgasten Compound gemäß Beispiel 1.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 170°C eingestellt. Als Formkörper wurde ein Plättchen 65 x 40 x 1 mm spritzgegossen.
Es konnten Plättchen frei von Schlieren mit einwandfreier glatter Oberfläche hergestellt werden. Die Plättchen ließen sich Problemlos entformen und sind geometriestabil.
An den erhaltenen Formteilen wird nach der in Beispiel 3 beschriebenen Methode mittels Potentiometrie die Alkalizahl bestimmt.
Als Ergebnis wurde eine Alkalizahl (mg KOH/g Polymer) von 22,3 erhalten. Zu Vergleichszwecke wurde ein nicht durch Spritzgießverarbeitung thermisch belastetes Polymer EUDRAGIT® RL 100 geprüft. Als Ergebnis wurde eine Alkalizahl von 22.9 erhalten. Der Wert mag zwar dicht an der Analysengenauigkeit liegen, deuten jedoch die Probleme der thermischen Zersetzung oberhalb 160°C an, Insbesondere bei Dauerbetrieb ist auch bei dieser Temperatur mit stärkerer Schädigung zu rechnen

### Beispiel 6 (erfindungsgemäß)

### Entgasung und Herstellung des Compounds

In einen 10l Mischbehälter aus Edelstahl werden 3,25 kg Granulat EUDRAGIT® RL 100 und 1,0 kg Talkum eingewogen und anschließend auf einem Taumelmischer 5 min gemischt.

Die hergestellte Mischung wurde auf einen Doppelschneckenextruder Typ 30.34 (Fa. Leistritz) gegeben, um einen erfindungsgemäßen Compound herzustellen. Die eingestellte Schmelzetemperatur betrug 140°C, die Schneckendrehzahl 120 /min. Nach einer Länge von 50 % der Gesamtlänge der Extruderschnecke wurde durch eine Öffnung in der Zylinderwand Triethylcitrat als Weichmacher über eine Membranpumpe in einer Menge zugegeben, die 20 % der Gesamtmenge entspricht. Nach einer Mischzone für die Homogenisierung der Mischung erfolgte die Entgasung über eine weitere Öffnung in der Zylinderwand. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt unter 0,1 % bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 140°C eingestellt. Als Formkörper wurde ein Plättchen 65 x 40 x 1 mm spritzgegossen.
Als Formkörper wurde ein Plättchen 65 x 40 x 1 mm spritzgegossen.
Es konnten Plättchen frei von Schlieren mit einwandfreier glatter Oberfläche hergestellt werden. Die Plättchen ließen sich problemlos entformen und sind geometriestabil.

### Beispiel 7 (erfindungsgemäß)

### Entgasung und Herstellung des Compounds

In einen Edelstahl-Mischer werden 3,25 kg Granulat EUDRAGIT® RL 100, und 3,25 kg Granulat EUDRAGIT® RS 100 und 0,03 kg Stearinsäure eingewogen und anschließend auf einem Taumelmischer 5 min gemischt.

Die hergestellte Mischung wurde auf einen Doppelschneckenextruder Typ 30.34 (Fa. Leistritz) gegeben, um einen erfindungsgemäßen Compound herzustellen. Die eingestellte Schmelzetemperatur betrug 140°C, die Schneckendrehzahl 120 /min. Nach einer Länge von 50 % der Gesamtlänge der Extruderschnecke wurde durch eine Öffnung in der Zylinderwand Triethylcitrat als Weichmacher über eine Membranpumpe in einer Menge zugegeben, die 10 % der Gesamtmenge entspricht. Nach einer Mischzone für die Homogenisierung der Mischung erfolgte die Entgasung über eine weitere Öffnung in der Zylinderwand. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt von 0,15 % bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 140°C eingestellt. Als Formkörper wurde ein Plättchen 65 x 40 x 1 mm spritzgegossen.

Als Formkörper wurde ein Plättchen 65 x 40 x 1 mm spritzgegossen. Es konnten Plättchen frei von Schlieren mit einwandfreier glatter Oberfläche hergestellt werden. Die Plättchen ließen sich problemlos entformen und sind geometriestabil.

### Beispiel 8 (erfindungsgemäß)

### Entgasung und Herstellung des Compounds

In einen 10l Mischbehälter aus Edelstahl werden 3,25 kg Granulat EUDRAGIT® RL 100 und 0,01 kg Stearinsäure eingewogen und anschließend auf einem Taumelmischer 5 min gemischt.

Die hergestellte Mischung wurde auf einen Doppelschneckenextruder Typ 30.34 (Fa. Leistritz) gegeben, um einen erfindungsgemäßen Compound herzustellen. Die eingestellte Schmelzetemperatur betrug 140°C, die Schneckendrehzahl 120 /min. Nach einer Länge von 50 % der Gesamtlänge der Extruderschnecke wurde durch eine Öffnung in der Zylinderwand Triethylcitrat als Weichmacher über eine Membranpumpe in einer Menge zugegeben, die 12,5 % der Gesamtmenge entspricht. Nach einer Mischzone für die Homogenisierung der Mischung erfolgte die Entgasung über eine weitere Öffnung in der Zylinderwand. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt von 0,13% bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 140°C eingestellt. Als Formkörper wurde ein Plättchen 65 x 40 x 1 mm spritzgegossen.

Es konnten Plättchen frei von Schlieren mit einwandfreier glatter Oberfläche hergestellt werden. Die Plättchen ließen sich problemlos entformen und sind geometriestabil.

### Beispiel 9 (erfindungsgemäß)

### Entgasung und Herstellung des Compounds

In einen 10l Mischbehälter aus Edelstahl werden 3,25 kg Granulat EUDRAGIT® RS 100 und 0,003 kg Stearinsäure eingewogen und anschließend auf einem Taumelmischer 5 min gemischt.

Die hergestellte Mischung wurde auf einen Doppelschneckenextruder Typ 30.34 (Fa. Leistritz) gegeben, um einen erfindungsgemäßen Compound herzustellen. Die eingestellte Schmelzetemperatur betrug 140°C, die Schneckendrehzahl 120 /min. Nach einer Länge von 50 % der Gesamtlänge der Extruderschnecke wurde durch eine Öffnung in der Zylinderwand Triethylcitrat als Weichmacher über eine Membranpumpe in einer Menge zugegeben, die 10 % der Gesamtmenge entspricht. Nach einer Mischzone für die Homogenisierung der Mischung erfolgte die Entgasung über eine weitere Öffnung in der Zylinderwand. Mittels der Düse am Extruderende wurden 4 Stränge geformt, über ein gekühltes Blech abgezogen und zu Granulat geschnitten. An dem erhaltenen Granulat wurde mittels Karl Fischer Titration ein Wassergehalt von 0,04% bestimmt.

### Spritzguss

Die erhaltene Mischung (Compound) wurde in den Trichter einer Spritzgießmaschine (Arburg Allrounder 250-125) gegeben und Formkörper spritzgegossen. An der Spritzgießmaschine wurden in Zone 3 und Zone 4 jedoch Temperaturen von 140°C eingestellt. Als Formkörper wurde ein Plättchen 65 x 40 x 1 mm spritzgegossen.

Es konnten Plättchen frei von Schlieren mit einwandfreier glatter Oberfläche hergestellt werden. Die Plättchen ließen sich problemlos entformen und sind geometriestabil.

## Patentansprüche

1. Verfahren zur Herstellung von Formkörpern mittels Spritzguss
mit den Verfahrensschritten
a) Herstellen einer Mischung, enthaltend zumindest ein (Meth)acrylatcopolymer, einen Weichmacher und ein Trockenstellmittel und/oder ein Trennmittel durch
Aufschmelzen und Mischen eines (Meth)acrylat-Copolymeren, das sich aus 85 bis 98 Gew.-% radikalisch polymerisierten C1- bis C4-Alkylestern der Acryl- oder der Methacrylsäure und 15 bis 2 Gew.-% (Meth)acrylat-Monomeren mit einer quaternären Ammoniumgruppe im Alkylrest zusammensetzt, mit 10 bis 25 Gew.-% eines Weichmachers, sowie 10 bis 50 Gew.-% eines Trockenstellmittel und/oder 0,1 bis 3 Gew.-% eines Trennmittels
b) Entgasen der Mischung bei Temperaturen von mindestens 120 °C, wodurch der Gehalt der niedrigsiedenden Bestandteile mit einem Dampfdruck mindestens 1,9 bar bei 120 °C auf höchstens 0,5 Gew.-% gesenkt wird und
c) Einspritzen der entgasten Mischung bei einer Temperatur von 80 bis 160 °C in die Form einer Spritzgussanlage und Entnahme des erhaltenen Formkörpers aus der Form.

2. Verfahren nach Anspruch 1, **dadurch gekennzeichnet, dass** die Mischung weitere pharmazeutisch übliche Additive oder Hilfsstoffe und/oder einen oder mehrere pharmazeutische Wirkstoffe enthält

3. Verfahren nach Anspruch 1 oder 2, **dadurch gekennzeichnet, dass** der Entgasungsschritt b) durch Extrusionstrocknung in einem Extruder mit Entgasungszone oder durch Entgasen in einer Spritzgussanlage mit vorgeschalteter Entgasungsöffnung erfolgt.

4. Spritzgegossener Formkörper, herstellbar in einem Verfahren nach einem oder mehreren der Ansprüche 1 bis 3.

5. Formkörper nach Anspruch 4, **dadurch gekennzeichnet, dass** er eine Schlagzähigkeit nach ISO 179 von mindestens 15 KJ/m² aufweist.

6. Formkörper nach Anspruch 4 oder 5 **dadurch gekennzeichnet, dass** ein oder mehrere pharmazeutische Wirkstoffe unmittelbar enthalten sind oder im Formkörper eingeschlossen sind.

7. Formkörper nach Anspruch 6, **dadurch gekennzeichnet, dass** es sich um eine Kapsel handelt, in der ein oder mehrere pharmazeutische Wirkstoffe eingeschlossen sind.

8. Formkörper nach einem oder mehreren der Ansprüche 4 bis 7, **dadurch gekennzeichnet, dass** der Formkörper Bestandteil einer größeren Arzneiform oder in dieser enthalten ist.

9. Formkörper nach einem oder mehreren der Ansprüche 4 bis 8, **dadurch gekennzeichnet, dass** ein oder mehrere pharmazeutische Wirkstoffe, die unmittelbar enthalten sind oder im Formkörper eingeschlossen sind, im tierischen oder menschlichen Magen-Darmtrakt retardierend abgegeben werden können.

10. Formkörper nach einem oder mehreren der Ansprüche 4 bis 9, **dadurch gekennzeichnet, dass** der pharmazeutische Wirkstoff Acetylsalicylsäure, Rantidin, Simvastatin, Enalapril, Fluoxetin, Amlodipin, Amoxicillin, Sertalin, Nifidipin, Ciprofloxacin, Acycolvir, Lovastatin,Epoetin, Paroxetin, Captopril, Nabumeton, Granisetron, Cimetidin, Ticarcillin, Triamteren, Hydrochlorothiazid, Verapamil, Paracetamol, Morphinderivate, Topotecan oder deren pharmazeutisch verwendeten Salze unmittelbar enthalten oder im Formkörper eingeschlossen ist.

## Revendications

1. Procédé pour la fabrication de corps moulés au moyen du moulage par injection
comprenant les étapes opératoires :
a) préparation d'un mélange contenant au moins un copolymère de (méth)acrylate, un plastifiant et un agent siccatif et/ou un agent de séparation par
fusion et mélangeage d'un copolymère de (méth)acrylate, qui se compose de 85 à 98 % en poids de C1 à C4-alkylesters de l'acide acrylique ou méthacrylique, polymérisés par voie radicalaire, et de 15 à 2 % en poids de monomères de (méth)acrylate avec un groupe ammonium quaternaire dans le radical alkyle, avec 10 à 25 % en poids d'un plastifiant, ainsi qu'avec 10 à 50 % en poids d'un agent siccatif et/ou 0,1 à 3 % en poids d'un agent de séparation,
b) dégazage du mélange à des températures d'au moins 120°C, à la suite duquel est abaissée à 0,5 % en poids au plus la teneur des constituants à bas point d'ébullition avec une tension de vapeur d'au moins 1,9 bars à 120°C et
c) injection du mélange dégazé à une température allant de 80 à 160°C dans le moule d'une installation de moulage par injection, puis extraction du corps moulé hors du moule.

2. Procédé selon la revendication 1, **caractérisé en ce que** le mélange contient d'autres additifs ou adjuvants pharmaceutiques usuels et/ou un ou plusieurs principes pharmaceutiques actifs.

3. Procédé selon la revendication 1 ou 2, **caractérisé en ce que** l'étape de dégazage b) est effectuée par séchage avec extrusion dans une extrudeuse ayant une zone de dégazage ou par dégazage dans une installation de moulage par injection ayant une ouverture de dégazage avec communication vers l'amont.

4. Corps moulé par injection, susceptible d'être fabriqué par un procédé selon l'une ou plusieurs des revendications 1 à 3.

5. Corps moulé selon la revendication 4, **caractérisé en ce qu'**il présente une résilience selon la norme ISO 179 égale à au moins 15 KJ/m².

6. Corps moulé selon la revendication 4 ou 5, **caractérisé en ce qu'**un ou plusieurs principes pharmaceutiques actifs y sont directement contenus ou sont encapsulés dans le corps moulé.

7. Corps moulé selon la revendication 6, **caractérisé en ce qu'**il s'agit d'une capsule, dans laquelle un ou plusieurs principes pharmaceutiques actifs sont encapsulés.

8. Corps moulé selon une ou plusieurs des revendications 4 à 7, **caractérisé en ce que** le corps moulé constitue une partie d'une forme médicamenteuse de grandeur plus importante ou est contenu dans celle-ci.

9. Corps moulé selon une ou plusieurs des revendications 4 à 8, **caractérisé en ce qu'**un ou plusieurs principes pharmaceutiques actifs, qui y sont directement contenus ou qui sont encapsulés dans le corps moulé, peuvent être administrés avec effet retard dans le tractus gastro-intestinal animal ou humain.

10. Corps moulé selon une ou plusieurs des revendications 4 à 9, **caractérisé en ce que** le principe pharmaceutique actif contient directement l'acide acétylsalicylique, la rantidine, la simvastatine, l'énalapril, la fluoxétine, l'amlodipine, l'amoxicilline, la sertaline, la nifidipine, la ciprofloxacine, l'acylclovir, la lovastatine, l'époetine, la paroxétine, le captopril, le nabuméton, le granisétron, la cimétidine, la ticarcilline, le triamtérène, l'hydrochlorothiazide, le vérapamil, le paracétamol, des dérivés de morphine, le topotécan ou leurs sels à utilisation pharmaceutique, ou se trouve être encapsulé dans le corps moulé.

## Claims

1. Process for producing mouldings by injection moulding,
the steps in the process being
a) preparation of a mixture, comprising at least one (meth)acrylate copolymer, a plasticizer and a dryer and/or a release agent via
melting and mixing of a (meth)acrylate copolymer composed of from 85 to 98% by weight of C1-C4-alkyl (meth)acrylates capable of free-radical polymerization and from 15 to 2% by weight of (meth)acrylate monomers having a quaternary ammonium group in the alkyl radical, with from 10 to 25% by weight of a plasticizer, and also from 10 to 50% by weight of a dryer and/or from 0.1 to 3% by weight of a release agent,
b) devolatilizing the mixture at temperatures of at least 120°C, thus reducing the content of the low-boiling constituents with a vapour pressure of at least 1.9 bar at 120°C to not more than 0.5% by weight, and
c) injecting the devolatilized mixture at a temperature of from 80 to 160°C into the mould of an injection moulding system and removing the resultant moulding from the mould.

2. Process according to Claim 1, **characterized in that** the mixture comprises other conventional pharmaceutical additives or auxiliaries and/or one or more active pharmaceutical ingredients.

3. Process according to Claim 1 or 2, **characterized in that** the devolatilizing step b) takes place via extrusion-drying in an extruder with a devolatilizing section, or by devolatilization in an injection moulding system with an upstream vent.

4. Injection moulding which can be produced in a process according to one or more of Claims 1 to 3.

5. Moulding according to Claim 4, **characterized in that** its impact strength to ISO 179 is at least 15 KJ/m².

6. Moulding according to Claim 4 or 5, **characterized in that** one or more active pharmaceutical ingredients are directly present or have been enclosed within the moulding.

7. Moulding according to Claim 6, **characterized in that** it is a capsule within which one or more active pharmaceutical ingredients have been enclosed.

8. Moulding according to one or more of Claims 4 to 7, **characterized in that** the moulding is a constituent of a larger drug form or is present within the same.

9. Moulding according to one or more of Claims 4 to 8, **characterized in that** one or more active pharmaceutical ingredients which are directly present or have been enclosed in the moulding can undergo delayed release in the gastrointestinal tract of an animal or human.

10. Moulding according to one or more of Claims 4 to 9, **characterized in that** the active pharmaceutical ingredient acetylsalicylic acid, rantidine, simvastatin, enalapril, fluoxetine, amlodipine, amoxicillin, sertaline, nifidipine, ciprofloxacin, acycolvir, lovastatin, epoetin, paroxetine, captopril, nabumetone, granisetron, cimetidine, ticarcillin, triamterene, hydrochlorothiazide, verapamil, paracetamol, morphine derivatives, topotecan, or pharmaceutically used salts of these is directly present or has been enclosed in the moulding.
